# EUROPEAN PATENT SPECIFICATION

(11) **EP 3 084 437 B1**
(45) Date of publication and mention of the grant of the patent: **28.02.2018**
(21) Application number: 14872376.0
(22) Date of filing: 17.12.2014
(51) Int. Cl.: G01N 33/53, G01N 33/567

(54) **DETECTION OF ENDOTHELIAL DISEASE**
DETEKTION VON ENDOTHELIALER ERKRANKUNG
DÉTECTION D'UNE MALADIE ENDOTHÉLIALE

(30) Priority: 18.12.2013 US 201361917783 P
(43) Date of publication of application: 26.10.2016
(73) Proprietor: Siemens Healthcare Diagnostics Inc., Tarrytown, NY 10591 (US)
(72) Inventor: PUGIA, Michael, Granger, IN 46540 (US); MARFURT, Karen L., Edwardsburg, MI 49112 (US)
(74) Representative: Schweitzer, Klaus
(86) International application number: PCT/US2014/071001
(87) International publication number: WO 2015/095426

(56) References cited:
- US-A1- 2002 028 204
- US-A1- 2004 110 241
- US-A1- 2004 265 311
- US-A1- 2005 244 897
- US-A1- 2011 294 206
- WOYWODT A ET AL: "Circulating endothelial cells as markers for ANCA-associated small-vessel vasculitis", THE LANCET, THE LANCET PUBLISHING GROUP, GB, vol. 361, no. 9353, 18 January 2003 (2003-01-18), pages 206-210, XP004765370, ISSN: 0140-6736, DOI: 10.1016/S0140-6736(03)12269-6
- BUTTHEP PUNNEE ET AL: "Endothelial injury and altered hemodynamics in thalassemia", CLINICAL HEMORHEOLOGY AND MICROCIRCULATION, vol. 31, no. 4, 2004, pages 287-293, XP9192313, ISSN: 1386-0291
- LEE ET AL.: 'Circulating endothelial cells, vonWillebrand factor, interleukin-6, and prognosis in patients with acute coronary syndromes.' BLOOD vol. 105, no. 2, 15 January 2005, pages 526 - 32, XP055290558

## Description

### BACKGROUND

The invention relates to methods of determining a level of endothelial disease in a subject. More particularly, the invention relates to methods of determining a potential of a mammalian subject for exhibiting premature coagulation.

Cellular analysis is important in medical applications, such as diagnosis of many diseases. However, many medical applications of cellular analysis require isolation of certain cells of interest, which typically represent only a small fraction of the analyzed sample. For example, circulating endothelial cells ("CEC") are of particular interest in the diagnosis of metastatic cancer and cardiovascular disorders. While CEC can be measured, the clinical value of such measurements is not clear. CEC have been shown to be present in patients with and without cardiovascular disease or cancer. Increase in CEC or change in morphology is not sufficient to predict myocardial infarction; thus, the use of such approaches does not have sufficient clinical value.

Measuring and maintaining hemostasis is necessary in prevention of thrombosis including, for example, blood clots such as deep vein thrombosis, disseminated intravascular coagulation (DIC), pulmonary embolism, myocardial thrombosis and stroke. Because hemostasis is a function that stops bleeding and protects the integrity of blood circulation, hemostasis is important in patients prone to coagulation due to injured blood vessels or a variety of conditions activating clotting such as, for example, atherosclerosis, sepsis, surgery, atrial fibrillation, congestive heart failure, uncontrolled hypertension, thrombocytopenia, auto immune diseases (e.g., Lupus), and other conditions leading to coagulation. Hemostasis is caused and/or impacted by a variety of clinical conditions and is not considered to be solely related to cardiovascular disease, infectious disease, cancer or other primary disease. Thrombosis is the formation of a blood clot inside a blood vessel, obstructing the flow of blood through the circulatory system. When a blood vessel is injured, the body uses platelets and fibrin to form a blood clot to prevent blood loss. Thrombosis is a pathological extension of normal hemostasis. Hemostasis is the arrest of hemorrhage in response to a vascular injury. Thrombosis is a formation of a blood clot in circulation. Thus, it is important to maintain hemostasis in patients whose potential for thrombosis is higher than normal.

Hemostasis is typically measured by detecting coagulation through the formation of a fibrin clot with clotting tests (e.g., prothrombin time), platelet aggregation or by the presence of activated proteases in plasma in the absence or presence of cofactors and inhibitors. However, such methods do not provide a complete picture because hemostasis is dependent on plasma proteins, platelets and vascular tissue (endothelium), which is at the site of injury and clot formation. Therefore, hemostasis information is the primary information needed for therapeutic action. Vascular tissue is not readily available for many patients. Cardiovascular health of patients has been assessed by subjecting isolated CEC to morphological analysis.

One major issue with CEC is that marker to cell type CEC are not specific to endothelial cells. CD105, CD51/61, CD73, S-ENDO/ MUC18, CD31/PECAM-1, CD36, AAMP, E and P selectin, and CD54 are also present on lymphocytes, monocytes and macrophages. Therefore, results can be altered by inflammation and infection. Additionally HEMCAM, Sca-1 and CD34 are expressed on hematopoietic precursor cells. Therefore, the measurement of cardiovascular diseases by CEC counts is not specific. Normal patients typically are counted as positives due to non-specific CEC, which occur without disease impact.

There is, therefore, a need to develop a non-morphological, minimally invasive method to analyze CEC to assess a patient's hemostasis potential or potential for endothelial disease and further to identify and treat a patient for premature coagulation.

### SUMMARY

Some examples in accordance with the principles described herein are directed to methods of determining a level of endothelial disease in a subject. The method comprises examining circulating endothelial cells from the subject for the presence of one or more coagulation factors and correlating an amount of circulating endothelial cells exhibiting one or more coagulation factors with the level of endothelial disease in the subject.

Some examples in accordance with the principles described herein are directed to methods of determining a potential of a mammalian subject for exhibiting premature coagulation. In the methods a concentration of circulating endothelial cells in a sample obtained from the mammalian subject is enhanced and the circulating endothelial cells are examined for the presence of a coagulation factor. The presence of the coagulation is correlated to the potential of the mammalian subject to exhibit premature coagulation.

Some examples in accordance with the principles described herein are directed to methods of identifying and treating a mammalian subject for premature coagulation. The method comprises enhancing a concentration of circulating endothelial cells in a sample obtained from the mammalian subject, examining the circulating endothelial cells for the presence of a coagulation factor, correlating the presence of the coagulation to the potential of the mammalian subject to exhibit premature coagulation, and administering to the mammalian subject anti-coagulation factor therapy.

### DETAILED DESCRIPTION OF SPECIFIC EMBODIMENTS

### General Discussion

Examples in accordance with the principles described herein involve a determination as to whether or not isolated CEC exhibit one or more coagulation factors on a surface thereof. The presence and/or amount of CEC having one or more coagulation factors may be used to measure the extent of endothelium dysfunction or damage and may be used also as an indication of premature coagulation (lack of hemostasis). Hemostasis of a patient is maintained by directing treatment to the coagulation factor(s) detected. Coagulation factors include, but are not limited to, factors I, II, III tissue factor, IV, V, VI, VII, VIII, IX, X, XI, XI, XII, thrombin, prothombin, antithromin, thrombomodulin, plasmin, plasminogen, urokinase, fibronectin, endothelial cell protein C, Von Willebrand factor (vWF), angiotensin-converting enzyme, fibrin, D-dimer, tissue plasminogen activator, protein Z related protease inhibitor, tissue factor pathway inhibitors, plasminogen activator inhibitor, bikunin, heparin cofactor II, cancer procoagulant, protein C, protein Z and protein S, for example.

Some examples in accordance with the principles described herein are directed to methods of determining a level of endothelial disease in a subject. Endothelial disease includes cardiovascular diseases, which include, but are not limited to, cardiac disease, vascular disease of the brain and kidney, and peripheral arterial disease, for example. Cardiac disease includes, but is not limited to, deep vein thrombosis, disseminated intravascular coagulation (DIC), pulmonary embolism, myocardial thrombosis, atherosclerosis, sepsis, surgery, atrial fibrillation (AF), congestive heart failure (CHF), uncontrolled hypertension (HTN), pulmonary hypertension, thrombocytopenia (TTP) and stroke, for example.

In some examples in accordance with the principles described herein, the method comprises enhancing a concentration of CEC in a sample obtained from the subject. The sample to be tested is usually biological. The phrase "biological sample" refers to any biological material such as, for example, body fluid, body tissue, body compounds and culture media. The sample may be a solid, semi-solid or a fluid (a liquid or a gas) from any biological source. In some examples the sample may be from the body of a subject including a body excretion, a body aspirant, a body excisant or a body extractant. The subject may be, for example, mammalian, reptilian, fish, plant, fungal, or bacterial. In some examples, the sample is from a mammal and in some examples the subject is a human. In some examples, the sample to be tested is a blood sample from a mammal such as, but not limited to, a human subject, for example. The blood sample is one that contains cells such as, for example, non-rare cells and rare cells. In some examples the blood sample is whole blood or plasma.

The presence and/or amount of CEC can be detected from other rare cells using markers characteristic of endothelial cells for cell typing. Endothelial Cell typing markers include, by way of illustration and not limitation, CD136, CD105/Endoglin, CD144/VE-cadherin, CD145, CD34, Cd41 CD136, CD34, CD90, CD31/PECAM-1, ESAM,VEGFR2/Flk-1, Tie-2, CD202b/TEK, CD56/NCAM, CD73/VAP-2, claudin 5, ZO-1 and vimentin, for example. In addition, Endothelial Cell typing markers that also provide the origin of the endothelial cell can be used as an additional or as the primary measure for CEC. Endothelial origin markers include, but are not limited to, CD36, HEMCAm for microvascular origin, SCA-1, or D34 for hematopoietic progenitor origin, VEGFR-2, NRP1, DII4, Notch, Ephrin B2, Connexin Cx37, Cx40 for arterial origin, VEGFR-3, NRP2, COUP-TFII, EphB4, Msr/Apj for venular origin, VEGR-3, NRP-2, LYVE-1, podoplanin, prox1, ephrin-B2, CCL21 for lymphatic origin, and LuECAM for lung venular origin, for example.

White blood cells (WBC) may be excluded from the assay by excluding WBC positive for one or more markers of cluster of differentiation (CD) that are specific for immune cells (also referred to as "cluster of designation" often abbreviated as CD). Examples include, by way of illustration and not limitation, markers such as CD45, CTLA-4, CD4, CD68 and/or CD8 present on white blood cells can be used to indicate that a cell is not a cancer cell. In a particular non-limiting example, CD45 antigen (also known as PTPRC, Protein tyrosine phosphatase receptor type C, and originally called leukocyte common antigen (all terms may be used herein interchangeably)) is useful in detecting all white blood cells. Additionally, CD45 can be used to differentiate the different types of white blood cells when combined with other CD markers. For example, granulocytes are indicated by CD45+, CD15+; monocytes are indicated by CD45+, CD14+; T lymphocytes are indicated by CD45+, CD3+; T helper cells are indicated by CD45+,CD3+, CD4+; cytotoxic T cells are indicated by CD45+,CD3+, CD8+; B lymphocytes are indicated by CD45+, CD19+ or CD45+, CD20+; thrombocytes are indicated by CD45+, CD61+; and natural killer cells are indicated by CD16+, CD56+, CD3-. Additionally, two commonly used CD molecules are CD4 and CD8, which are, in general, used as markers for helper and cytotoxic T cells, respectively. These molecules are defined in combination with CD3+, as some other leukocytes also express these CD molecules (some macrophages express low levels of CD4; dendritic cells express high levels of CD8).

Rare cells are those cells that are present in a sample in relatively small quantities when compared to the amount of non-rare cells in a sample. In some examples, the rare cells are present in an amount of about 10⁻⁸ % to about 10⁻² % by weight of a total cell population in a sample suspected of containing the rare cells. CEC are included in the category of rare cells.

Non-rare cells are those cells that are present in relatively large amounts when compared to the amount of rare cells such as CEC in a sample. In some examples, the non-rare cells are at least about 10 times, or at least about 10² times, or at least about 10³ times, or at least about 10⁴ times, or at least about 10⁵ times, or at least about 10⁶ times, or at least about 10⁷ times, or at least about 10⁸ times greater than the amount of the rare cells in the total cell population in a sample suspected of containing non-rare cells and rare cells. The non-rare cells may be, but are not limited to, white blood cells (WBC), platelets, and red blood cells (RBC), for example.

Enhancement of the concentration of CEC (or isolation of CEC) in a sample from a subject may be achieved by filtration using one or more of the following forces: centrifugation, pressure, vacuum, capillary hydrostatic forces, ultracentrifugation, differential thermal gradient and electrophoretic forces, for example. Filtration involves contacting the sample with a porous matrix usually under vacuum pressure. Filtration techniques include, but are not limited to, microfiltration, ultrafiltration, or cross-flow filtration, for example. In some examples, the porous matrix may be part of a filtration module where the porous matrix is part of an assembly for convenient use during filtration ("porous matrix filtration assembly"). The porous matrix can be a membrane, film, or microfluidic structure that has pores or obstacles through which liquid with cells flows across or through.

One example of a porous matrix filtration assembly, by way of illustration and not limitation, is described by Friedrich, et al., in U.S. Patent Application Publication No. 2012/0315664 ('''664 patent application publication"). The '664 patent application publication discloses an assembly and method for the filtration of a liquid. A supporting body is designed in a recess of a carrier and a filter membrane lies flat on the supporting body. The filter membrane and the supporting body are designed to be permeable to liquids and thus serve as filters, in particular for filtering rare cells from blood. As a result of the filter membrane lying level on the supporting body, the filtration residue can be particularly well examined microscopically.

In some examples samples are collected from the body of a subject into a suitable container such as, but not limited to, a bag, a bottle, a needle or a VACUTAINER® container, for example. The container may contain a collection medium into which the sample is delivered. The collection medium is usually a dry medium and may comprise an amount of platelet deactivation agent effective to achieve deactivation of platelets in the blood sample when mixed with the blood sample. The collection medium may also comprise other agents such as, for example, an anti-coagulant, a fixing agent, or a fibrin-formation-arresting agent, each of which are present in an amount sufficient to achieve the desired function of the agents. In some examples, a sample may be treated to preferentially agglutinate CEC over non-rare cells to facilitate separation of CEC from non-rare cells during filtration.

The porous matrix is a solid or semi-solid material and may be comprised of an organic or inorganic, water insoluble material. The porous matrix can have any of a number of shapes such as, for example, tubular (e.g., hollow fiber, spiral wound, and hollow fine fiber), track-etched, or planar or flat surface (e.g., strip, disk, film, membrane, and plate). The matrix may be fabricated from a wide variety of materials, which may be naturally occurring or synthetic, polymeric or non-polymeric, fibrous or non-fibrous. Examples, by way of illustration and not limitation, of such materials for fabricating a porous matrix include cellulose (including paper), nitrocellulose, cellulose acetate, polycarbonate, poly (vinyl chloride), polyacrylamide, polyacrylate, polyethylene, polypropylene, poly-(4-methylbutene), polystyrene, polymethacrylate, poly(ethylene terephthalate), nylon, and poly(vinyl butyrate), ceramic material, metallic material, for example, either used by themselves or in conjunction with one another and/or with other materials.

The size of the pores of the porous matrix is that which is sufficient to preferentially retain CEC while allowing the passage of other cells including non-rare cells through the pores. The size of the pores of the porous matrix is dependent on the size of the CEC and the non-rare cells, the pressure applied to the sample such as a blood sample, for example. In some examples the average size of the pores of the porous matrix is about 1 µm to about 100 µm, or about 1 µm to about 75 µm, or about 1 µm to about 50 µm, or about 1 µm to about 20 µm, or about 1 µm to about 10 µm, or about 5 µm to about 100 µm, or about 5 µm to about 75 µm, or about 5 µm to about 50 µm, or about 5 µm to about 20 µm, or about 5 µm to about 10 µm, for example. The density of pores in the porous matrix is about 1% to about 80%, or about 10% to about 80%, or about 20% to about 80%, or about 50% to about 80%, or about 20% to about 70%, for example.

In some examples in accordance with the principles described herein, pressure is applied to the sample on the porous matrix to facilitate passage of non-rare cells through the membrane. The term "pressure" refers to pressure differences from normal atmospheric pressure and can be either positive pressure (increase in pressure relative to normal atmospheric pressure) or negative pressure (vacuum) (decrease in pressure relative to normal atmospheric pressure). The level of pressure applied is dependent on one or more of the nature and size of the non-rare cells, the size of the CEC, the nature of the porous matrix, and the size of the pores of the porous matrix, for example. In some examples, the level of positive pressure applied is about 1 millibar to about 500 millibar, or about 1 millibar to about 400 millibar, or about 1 millibar to about 300 millibar, or about 1 millibar to about 200 millibar, or about 1 millibar to about 100 millibar, or about 1 millibar to about 50 millibar, or about 1 millibar to about 30 millibar, or about 1 millibar to about 25 millibar, or about 1 millibar to about 20 millibar, or about 1 millibar to about 15 millibar, or about 1 millibar to about 10 millibar, or about 5 millibar to about 30 millibar, or about 5 millibar to about 25 millibar, or about 5 millibar to about 20 millibar, or about 5 millibar to about 15 millibar, or about 5 millibar to about 10 millibar, for example. The level of negative pressure (vacuum) applied is the negative of the above ranges.

In some examples the pressure applied to the sample on the porous matrix is an oscillating pressure, which means that the pressure is applied intermittently at regular or irregular intervals, which may be, for example, about 1 second to about 600 seconds, or about 1 second to about 500 seconds, or about 1 second to about 250 seconds, or about 1 second to about 100 seconds, or about 1 second to about 50 seconds, or about 10 seconds to about 600 seconds, or about 10 seconds to about 500 seconds, or about 10 seconds to about 250 seconds, or about 10 seconds to about 100 seconds, or about 10 seconds to about 50 seconds, or about 100 seconds to about 600 seconds, or about 100 seconds to about 500 seconds, or about 100 seconds to about 250 seconds, for example. In this approach, pressure is oscillated at about 0 millibar to about 10 millibar, or about 1 millibar to about 10 millibar, or about 1 millibar to about 7.5 millibar, or about 1 millibar to about 5.0 millibar, or about 1 millibar to about 2.5 millibar, for example, during some or all of the application of pressure to the blood sample. Oscillating pressure is achieved using an on-off switch, for example, and may be conducted automatically or manually. High pressure drops are allowable depending on one or more of reservoir volume, sample volume and filtration rate.

As mentioned above, the sample is contacted with a porous matrix such that CEC are preferentially retained on the porous matrix and non-rare cells pass through the porous matrix. In some examples in accordance with the principles described herein, the sample is diluted with a dilution medium prior to contact with the porous matrix. In some examples, the dilution medium is an aqueous medium, which may be buffered. The pH for an aqueous buffered medium is usually a moderate pH. In some examples the pH of the dilution medium is about 5 to about 8, or about 6 to about 8, or about 7 to about 8, or about 5 to about 7, or about 6 to about 7, or physiological pH, for example. Various buffers may be used to achieve the desired pH and maintain the pH during any incubation period. Illustrative buffers include, but are not limited to, borate, phosphate (e.g., phosphate buffered saline), carbonate, TRIS, barbital, PIPES, HEPES, MES, ACES, MOPS, and BICINE, for example.

The amount of dilution medium combined with the sample is dependent on one or more of a number of factors such as, for example, the nature of the porous matrix and the nature of the sample. In some examples in accordance with the principles described herein, the amount of dilution medium is about 5 mL to about 100 mL, or about 5 mL to about 80 mL, or about 5 mL to about 60 mL, or about 5 mL to about 50 mL, or about 5 mL to about 30 mL, or about 5 mL to about 20 mL, or about 5 mL to about 10 mL, or about 10 mL to about 100 mL, or about 10 mL to about 80 mL, or about 10 mL to about 60 mL, or about 10 mL to about 50 mL, or about 10 mL to about 30 mL, or about 10 mL to about 20 mL, or about 20 mL to about 100 mL, or about 20 mL to about 80 mL, or about 20 mL to about 60 mL, or about 20 mL to about 50 mL, or about 20 mL to about 30 mL, for example, based on 10 mL of the sample.

Contact of the sample with the porous matrix is continued for a period of time sufficient to achieve an enrichment of the CEC to non-rare cells on the porous matrix. The period of time is dependent on one or more of the nature and size of the non-rare cells, the size of the CEC, the nature of the porous matrix, the size of the pores of the porous matrix, the level of pressure applied to the blood sample on the porous matrix, the volume to be filtered, the surface area of the filter, for example. In some examples, the period of contact is about 1 minute to about 1 hour, about 5 minutes to about 1 hour, or about 5 minutes to about 45 minutes, or about 5 minutes to about 30 minutes, or about 5 minutes to about 20 minutes, or about 5 minutes to about 10 minutes, or about 10 minutes to about 1 hour, or about 10 minutes to about 45 minutes, or about 10 minutes to about 30 minutes, or about 10 minutes to about 20 minutes, for example.

In some examples a desired increase in the ratio of CEC to non-rare cells in a sample suspected of containing CEC and non-rare cells is at least about 10 fold, or at least about 20 fold, or at least about 50 fold, or at least about 75 fold, or at least about 100 fold over the ratio of the CEC to the non-rare cells in the original sample. In some examples, the increase of the ratio of CEC to non-rare cells that is desired is about 10 fold to about 200 fold, or about 10 fold to about 150 fold, or about 10 fold to about 100 fold, or about 25 fold to about 100 fold, or about 50 fold to about 100 fold, for example.

The concentrated or isolated CEC are next examined for the presence of one or more coagulation factors. In some examples in accordance with the principles described herein, such an examination involves an assay that uses a binding partner for a specific coagulation factor.

Any suitable assay may be employed for determining a presence and/or amount of coagulation factor present on the CEC. The assays are conducted by combining enhanced or isolate cells with reagents for identifying one or more coagulation factors, which include binding partner for the coagulation factor. The binding partner is an agent that specifically recognizes and/or binds to a coagulation factor on the CEC. The nature of the reagents is dependent on the particular type of assay to be performed. In general, the assay is a method for the determination of a coagulation factor on a CEC. The assay may be an immunoassay or a non-immunoassay. The assay may be carried out concomitantly with the filtration or may be carried out after the filtration. Various assay methods are discussed below by way of illustration and not limitation.

In some examples the reagents comprise, as a binding partner for a specific coagulation factor, at least one antibody specific for a coagulation factor that may be present on the CEC. This assay is generally referred to as an immunoassay as distinguished from assays that do not utilize an antibody, which are referred to as non-immunoassays. By the phrase "antibody for a coagulation factor" is meant an antibody that binds specifically to the coagulation factor and does not bind to any significant degree to other substances that would distort the analysis for the particular coagulation factor.

Antibodies specific for a coagulation factor for use in immunoassays to identify a particular coagulation factor can be monoclonal or polyclonal. Such antibodies can be prepared by techniques that are well known in the art such as immunization of a host and collection of sera (polyclonal) or by preparing continuous hybrid cell lines and collecting the secreted protein (monoclonal) or by cloning and expressing nucleotide sequences or mutagenized versions thereof coding at least for the amino acid sequences required for specific binding of natural antibodies.

Antibodies include a complete immunoglobulin or fragment thereof, which immunoglobulins include the various classes and isotypes, such as IgA, IgD, IgE, IgG1, IgG2a, IgG2b and IgG3, and IgM, for example. Fragments thereof include Fab, Fv and F(ab')₂, and Fab', for example. In addition, aggregates, polymers, and conjugates of immunoglobulins or their fragments can be used where appropriate so long as binding affinity for a particular molecule is maintained.

Other reagents are included in the assay medium depending on the nature of the assay to be conducted. Such assays usually involve reactions between binding partners such as a coagulation factor on a CEC and a corresponding antibody or the binding between an antibody and a corresponding binding partner such as a second antibody that binds to the first antibody. The antibody and the coagulation factor are members of a specific binding pair, which comprises two different molecules, each of which having an area on the surface or in a cavity that specifically binds to and is thereby defined as complementary with a particular spatial and polar organization of the other molecule. The members of the specific binding pair will usually be members of an immunological pair such as antigen-antibody and hapten-antibody, although other specific binding pairs include, for example, biotin-avidin, hormones-hormone receptors, enzyme-substrate, nucleic acid duplexes, IgG-protein A, and polynucleotide pairs such as DNA-DNA, DNA-RNA.

As discussed above, specific binding involves the specific recognition of one of two different molecules for the other compared to substantially less recognition of other molecules. On the other hand, non-specific binding involves non-covalent binding between molecules that is relatively independent of specific surface structures. Non-specific binding may result from several factors including hydrophobic interactions between molecules. In many embodiments of assays for a particular coagulation factor, preferred binding partners are antibodies and the assays are referred to as immunoassays.

The immunoassays may involve labeled or non-labeled reagents. Immunoassays involving non-labeled reagents usually comprise the formation of relatively large complexes involving one or more antibodies. Such assays include, for example, immunoprecipitin and agglutination methods and corresponding light scattering techniques such as, e.g., nephelometry and turbidimetry, for the detection of antibody complexes. Labeled immunoassays include enzyme immunoassays, fluorescent-labeled immunoassays, fluorescence polarization immunoassays, radioimmunoassay, inhibition assay, induced luminescence, and fluorescent oxygen channeling assay, for example.

The assays can be performed either without separation (homogeneous) or with separation (heterogeneous) of any of the assay components or products. Homogeneous immunoassays include, but are not limited to, immunocytochemistry techniques, the direct fluorescent antibody test or the direct immunofluorescence test, homogeneous enzyme immunoassays including the EMIT® assay disclosed in U.S. Patent No. 3,817,837; immunofluorescence methods such as those disclosed in U.S. Patent No. 3,996,345; enzyme channeling immunoassays such as those disclosed in U.S. Patent No. 4,233,402; and the fluorescence polarization immunoassay as disclosed, for example, in U.S. Patent No. 5,354,693; for example.

Other enzyme immunoassays include the enzyme modulate mediated immunoassay; the substrate labeled fluorescence immunoassay; the combined enzyme donor immunoassays; homogeneous particle labeled immunoassays such as particle enhanced turbidimetric inhibition immunoassays and the particle enhanced turbidimetric immunoassay; for example. Other assays include the sol particle immunoassay; the disperse dye immunoassay; the metalloimmunoassay; the enzyme membrane immunoassays; luminoimmunoassays; acridinium ester label immunoassays using paramagnetic particles as a solid phase; for example. Other types of assays include immunosensor assays involving the monitoring of the changes in one or more of the optical, acoustic and electrical properties of an antibody-immobilized surface upon the binding of a hydrophobic drug. Such assays include, for example, optical immunosensor assays, acoustic immunosensor assays, semiconductor immunosensor assays, electrochemical transducer immunosensor assays, potentiometric immunosensor assays, amperometric electrode assays, and the like.

In many of the assays discussed herein for determination of a coagulation factor, a label is employed; the label is usually part of a signal producing system. The nature of the label is dependent on the particular assay format. A signal producing system usually includes one or more components, at least one component being a detectable label, which generates a detectable signal that relates to the amount of bound and/or unbound label, i.e., the amount of label bound or not bound to the analyte being detected or to an agent that reflects the amount of the analyte to be detected. The label is any molecule that produces or can be induced to produce a signal, and may be, for example, a fluorescer, a radiolabel, an enzyme, a chemiluminescer or a photosensitizer. Thus, the signal is detected and/or measured by detecting enzyme activity, luminescence, light absorbance or radioactivity, depending on the nature of the label.

Suitable labels include, by way of illustration and not limitation, dyes; fluorescers, such as fluorescein, isothiocyanate, rhodamine compounds, phycoerythrin, phycocyanin, allophycocyanin, o-phthaldehyde, and fluorescamine; enzymes such as alkaline phosphatase, glucose-6-phosphate dehydrogenase ("G6PDH"), β-galatosidase, and horseradish peroxidase; ribozyme; a substrate for a replicase such as QB replicase; promoters; complexes such as those prepared from CdSe and ZnS present in semiconductor nanocrystals known as Quantum dots; chemiluminescers such as isoluminol and acridinium esters, for example; sensitizers; coenzymes; enzyme substrates; radiolabels such as ¹²⁵l, ¹³¹l, ¹⁴C, ³H, ⁵⁷Co and ⁷⁵Se; particles such as latex particles, carbon particles, metal particles including magnetic particles, e.g., chromium dioxide (CrO₂) particles, and the like; metal sol; crystallite; liposomes; cells, etc., which may be further labeled with a dye, catalyst or other detectable group.

The label can directly produce a signal and, therefore, additional components are not required to produce a signal. Numerous organic molecules, for example fluorescers, are able to absorb ultraviolet and visible light, where the light absorption transfers energy to these molecules and elevates them to an excited energy state. This absorbed energy is then dissipated by emission of light at a second wavelength. Other labels that directly produce a signal include radioactive isotopes and dyes.

Alternately, the label may need other components to produce a signal, and the signal producing system would then include all the components required to produce a measurable signal. Such other components may include substrates, coenzymes, enhancers, additional enzymes, substances that react with enzymic products, catalysts, activators, cofactors, inhibitors, scavengers, metal ions, and a specific binding substance required for binding of signal generating substances. A detailed discussion of suitable signal producing systems can be found in U.S. Patent No. 5,185,243, columns 11-13.

The label or other signal producing system members can be bound to a support or become bound to a molecule such as a cell that is disposed on a support. Cells may be bound to a solid support in any manner known in the art, provided only that the binding does not substantially interfere with the ability of a coagulation factor on the cell to bind with an antibody. In some embodiments, the cells may be coated or covalently bound directly to the solid support. Linking groups may also be used to covalently couple the solid support and the cells. Other methods of binding the cells are also possible. For instance, a solid support may have a coating of a binder for a small molecule such as, for example, avidin or an antibody, and a small molecule such as, e.g., biotin, hapten, etc., can be bound to the cells or vice versa. The binding of components to the surface of a support may be direct or indirect, covalent or non-covalent and can be accomplished by well-known techniques, commonly available in the literature.

The support may be comprised of an organic or inorganic, solid or fluid, water insoluble material, which may be transparent or partially transparent. The support can have any of a number of shapes, such as particle, including bead, film, membrane, tube, well, strip, rod, planar surfaces (such as, e.g., sheet, plate and slide), and fiber, for example. Depending on the type of assay, the support may or may not be suspendable in the medium in which it is employed. Examples of suspendable supports are polymeric materials such as latex; lipid bilayers or liposomes; oil droplets, and metallic supports such as, e.g., magnetic particles; for example. Other support compositions include polymers, such as nitrocellulose, cellulose acetate, poly (vinyl chloride), polyacrylamide, polyacrylate, polyethylene, polypropylene, poly(4-methylbutene), polystyrene, polymethacrylate, poly(ethylene terephthalate), nylon, and poly(vinyl butyrate), either used by themselves or in conjunction with other materials.

The label and/or other signal producing system member may be bound to a member of a specific binding pair or another molecule. For example, the label can be bound covalently to a member of a specific binding pair such as, for example, an antibody, a receptor for an antibody, or a receptor that is capable of binding to a small molecule conjugated to an antibody. A receptor is a molecule capable of binding specifically to another molecule. Bonding of the label to the specific binding pair member may be accomplished by chemical reactions that result in replacing a hydrogen atom of the label with a bond to the specific binding pair member or may include a linking group between the label and the specific binding pair member. Other signal producing system members may also be bound covalently to specific binding pair members. For example, two signal producing system members such as a fluorescer and quencher can each be bound to a different antibody that forms a specific complex with the coagulation factor. Formation of the complex brings the fluorescer and quencher in close proximity, thus permitting the quencher to interact with the fluorescer to produce a signal.

The assays discussed above are normally carried out in an aqueous buffered medium at a moderate pH, generally that which provides optimum assay sensitivity. The pH for the assay medium will usually be in the range of about 4 to about 11, or in the range of about 5 to about 10, or in the range of about 6.5 to about 9.5. The pH will usually be a compromise between optimum binding of the binding members of any specific binding pairs, and the pH optimum for other reagents of the assay such as members of a signal producing system, for example. Various buffers may be used to achieve the desired pH and maintain the pH during the incubation period. Illustrative buffers include borate, phosphate, carbonate, TRIS, barbital, PIPES, HEPES, MES, ACES, MOPS, BICINE, and the like. Various ancillary materials may be employed in the assay methods. For example, in addition to buffers and preservatives, the medium may comprise stabilizers for the medium and for the reagents employed. In some embodiments, in addition to these additives, proteins may be included, such as albumins; quaternary ammonium salts; polyanions such as dextran sulfate; and binding enhancers, for example. All of the above materials are present in a concentration or amount sufficient to achieve the desired effect or function.

One or more incubation periods may be applied to the medium at one or more intervals including any intervals between additions of various reagents mentioned above. The medium is usually incubated at a temperature and for a time sufficient for binding of various components of the reagents to occur. Moderate temperatures are normally employed for carrying out the method and usually constant temperature, preferably, room temperature, during the period of the measurement. Incubation temperatures normally range from about 5°C to about 99°C or from about 15°C to about 70°C, or about 20°C to about 45°C, for example. The time period for the incubation is about 0.2 seconds to about 24 hours, or about 1 second to about 6 hours, or about 2 seconds to about 1 hour, or about 1 to about 15 minutes, for example. The time period depends on the temperature of the medium and the rate of binding of the various reagents. Temperatures during measurements will generally range from about 10°C to about 50°C or from about 15°C to about 40°C.

The concentrations of the various reagents in the assay medium will generally be determined by the concentration range of interest of the coagulation factor, the nature of the assay, the antibody affinity and avidity and antibody fragmentation, for example. However, the final concentration of each of the reagents is normally determined empirically to optimize the sensitivity of the assay over the range. Considerations such as the nature of a signal producing system and the nature of the coagulation factor normally determine the concentrations of the various reagents.

While the order of addition may be varied widely, there will be certain preferences depending on the nature of the assay. The simplest order of addition is to add all the materials simultaneously and determine the effect that the assay medium has on the signal as in a homogeneous assay. Alternatively, the reagents can be combined sequentially. Optionally, an incubation step may be involved subsequent to each addition as discussed above. The length of the incubation period is that which is sufficient to accomplish the desired function.

Specific embodiments of assays that may be employed to identify one or more coagulation factors on CEC in accordance with the principles described herein are discussed below by way of illustration and not limitation.

In one example, an immunocytochemistry technique is employed to determine whether or not a particular coagulation factor is present on the CEC. The isolated CEC preparation is placed on a solid support, which may be, for example, a microscope slide. The isolated CEC preparation may be removed from a filter membrane, for example, and placed on a solid support for examination or the filter membrane itself may be placed on the solid support. The CEC preparation may be treated to fix the cells and/or to permeabilize the cells, if desired.

Fixation of the CEC immobilizes the cells and preserves cell structure and maintains the cells in a condition that closely resembles the cells in an *in vivo*-like condition and one in which the coagulation factors of interest are able to be recognized by a specific antibody. The amount of fixative employed is that which preserves the cells but does not lead to erroneous results in a subsequent assay. The amount of fixative depends on one or more of the nature of the fixative and the CEC, for example. In some examples, the amount of fixative is about 0.05% to about 0.15% or about 0.05% to about 0.10%, or about 0.10% to about 0.15%, for example, by volume of the blood sample. Agents for carrying out fixation of the rare cells include, but are not limited to, cross-linking agents such as, for example, an aldehyde reagent (such as, e.g., formaldehyde, glutaraldehyde, and paraformaldehyde,); an alcohol (such as, e.g., C1-C5 alcohols such as methanol, ethanol and isopropanol); a ketone (such as a C3-C5 ketone such as acetone); for example. The designations C1-C5 or C3-C5 refer to the number of carbon atoms in the alcohol or ketone. One or more washing steps may be carried out on the fixed cells using a buffered aqueous medium.

If necessary after fixation, the CEC preparation may be subjected to permeabilization. In some instances, a fixation agent such as, for example, an alcohol (e.g., methanol or ethanol) or a ketone (e.g., acetone) also results in permeabilization and no additional permeabilization step is necessary. Permeabilization provides access through the cell membrane to coagulation factors of interest. The amount of permeabilization agent employed is that which disrupts the cell membrane and permits access to the coagulation factors. The amount of permeabilization agent depends on one or more of the nature of the permeabilization agent and the amount of CEC, for example. In some examples, the amount of permeabilization agent is about 0.1% to about 0.5%, or about 0.1% to about 0.4%, or about 0.1% to about 0.3%, or about 0.1% to about 0.2%, or about 0.2% to about 0.5%, or about 0.2% to about 0.4%, or about 0.2% to about 0.3%, for example. Agents for carrying out permeabilization of the rare cells include, but are not limited to, an alcohol (such as, e.g., C1-C5 alcohols such as methanol and ethanol); a ketone (such as a C3-C5 ketone such as acetone); a detergent (such as, e.g., saponin, TRITON® X-100, and TWEEN®-20); for example. One or more washing steps may be carried out on the permeabilized cells using a buffered aqueous medium.

In the immunocytochemistry technique, a labeled antibody specific for a coagulation factor on a CEC is employed for each suspected different coagulation factor in the CEC preparation. The labels employed are fluorescent labels and a different fluorescent label is employed for each different coagulation factor such that multiple fluorescent-labeled antibodies may be employed in any one assay conducted on an isolated CEC preparation.

Following fixation and permeabilized, the CEC preparation is contacted with an aqueous medium containing one or more labeled antibodies as described above. The aqueous medium may be an assay medium as described above and the amount of each labeled antibody is that which is sufficient to identify each of the coagulation factors that may be of interest. In some examples, the amount of each labeled antibody is in excess of the suspected amount of the coagulation factors. The CEC are incubated with the labeled antibodies under conditions that permit binding of the labeled antibodies to their respective coagulation factors. Such conditions are discussed above regarding assays in general. After incubation, the CEC preparation is subjected to one or more washing steps using an aqueous buffered medium to remove unbound labeled antibodies.

A fluorescent DNA stain such as, for example, 4',6-diamidino-2-phenylindole, propidium iodide, ethidium bromide, SYBR® Green I, VISTRA™ GREEN, SYTO® GREEN, SYBR® Gold, YO-PRO-1™, TOTO-3™, TO-PRO-3™, NUCLEAR-ID™ Red, or Hoechst dye, is employed to enhance contrast in CEC preparation image during microscopic examination. After staining, one or more washing steps may be carried out on the cells using a buffered aqueous medium. The cells are then examined using a fluorescent microscope and each of the different fluorescent labels is used in the direct detection of a respective coagulation factor in the CEC preparation.

Alternatively, in the above procedure unlabeled antibodies may be employed and the respective antibodies are detected indirectly employing a specific binding member such as, for example, anti-species antibody with a label. The respective labels of the specific binding members are detected by appropriate means.

Alternatively, in the above procedures antibodies may be employed where the respective antibodies are indirectly detected with a second antibody labeled with an enzyme or directly labeled with an enzyme in an enzyme amplification method. A respective enzyme such as, for example, horse radish peroxidase (HRP) is detected by appropriate means. For example by enzymatic generation of a respective fluorescent, optical or chemiluminescent signal, a respective enzyme label is generated to covalently attached dye to protein tyrosine groups in the presence of HRP (such as, for example, Tyramide Signal Amplification (TSA™), Perkin Elmer), for example. The specific binding members may be, for example, an antibody specific for each of the respective unlabeled antibodies used for binding to a respective coagulation factor.

In some examples in accordance with the principles described herein, one of the immunoassay methods, such as, for example, an immunocytochemistry method, is carried out in conjunction with the use of a porous matrix filtration assembly described above. In one example, by way of illustration and not limitation, the porous matrix filtration assembly described in the '664 patent application publication is employed. CEC retained on the filter of the assembly are examined using an immunoassay method such as, for example, the immunocytochemistry method discussed more fully above. In a particular example, a sample to be tested for CEC cells is subjected to filtration employing the porous matrix filtration assembly of the '664 patent application publication. CEC concentrated on a surface of the porous matrix are examined by exposing the CEC to one or more labeled antibodies, each of which are specific for a coagulation factor on a CEC. The labels employed are fluorescent labels and a different fluorescent label is employed for each different coagulation factor such that multiple fluorescent-labeled antibodies may be employed in any one assay conducted on an isolated CEC preparation. Following fixation and permeabilized, the CEC preparation is contacted with an aqueous medium containing one or more labeled antibodies as described above. The CEC are incubated with the labeled antibodies under conditions that permit binding of the labeled antibodies to their respective coagulation factors. After incubation, the CEC preparation is subjected to one or more washing steps using an aqueous buffered medium to remove unbound labeled antibodies. A fluorescent DNA stain, as discussed above, is employed to enhance contrast in the CEC preparation image during microscopic examination. After staining, one or more washing steps may be carried out on the cells using a buffered aqueous medium.

The cells are then examined using a fluorescent microscope and each of the different fluorescent labels is used in the direct detection of a respective coagulation factor in the CEC preparation. In examples in accordance with the discussion above, lower backgrounds were surprisingly observed so that the weaker coagulation factor signal (lower affinity immunoassay) can be seen as separate signal from background noise in the endothelial cell identification marker signal. This is an unexpected advantage realized in examples of methods for CEC analysis employing porous matrix filtration techniques and immunocytochemistry techniques.

The measurement of an amount of signal obtained in any of the above assay methods for CEC comprising one or more coagulation factors is correlated with the level of endothelial disease in the subject. This measurement is explained more fully as follows: To first determine if the cell of interest is an endothelial cell using an endothelial cell marker with separate distinct signal and by comparison to an immune cell marker in a second separate distinct signal. The CEC cells are counted when the CEC is above a threshold and the WBC signal is below a threshold. The threshold is defined as the point at which the signal is detectable over the noise. The noise in this case is the background signal on the filtration matrix. An additional third separate distinct signal for the coagulation factor marker is measured for each CEC. The CEC cells with a measured coagulation factor signal above the threshold are counted as activated CEC. The filtration process allows all three signals to be measured simultaneously. A separate signal threshold is selected for each determination of whether a cell is CEC, WBC or whether a CEC has a coagulation factor. The WBC signal threshold is established using a negative control with WBC. The CEC and coagulation factor signal thresholds are determined using a positive control with WBC and cultured endothelial cells that are fixed with a coagulation factor. Signals below the threshold are set to zero. The unknown sample is run and only the coagulation factor signals above the threshold measured on cells that are CEC positive and WBC negative cells.

The above methods may also be employed to determine a potential of a mammalian subject for exhibiting premature coagulation or thrombosis. The signal obtained in any of the above assay methods for CEC comprising one or more coagulation factors is correlated to the potential of a mammalian subject for exhibiting premature coagulation or thrombosis by measuring the CEC and WBC signals identifying the cells above the CEC threshold and below the WBC threshold. The coagulation factor signal on the CEC is then measured if above the positive threshold. The numbers of CEC are counted for each patient. If the signal for the coagulation factor is above the threshold for the CEC, the cell is considered activated. The higher the increase in the coagulation factor signal increase above the threshold, the greater is the activation for the CEC. The higher the number of CEC, the number of activated CEC and the extent of activation, the greater is the correlation the subject exhibiting premature coagulation.

Some examples in accordance with the principles described herein are directed to methods of identifying and treating a mammalian subject for premature coagulation. The method comprises enhancing a concentration of circulating endothelial cells in a sample obtained from the mammalian subject, examining the circulating endothelial cells for the presence of a coagulation factor, correlating the presence of the coagulation to the potential of the mammalian subject to exhibit premature coagulation, and administering to the mammalian subject anti-coagulation factor therapy.

Examples of methods in accordance with the principles described herein offer significant advantages with regard to therapy for subjects identified as having a potential for premature coagulation. As mentioned above, anti-coagulation factor therapy may be employed, which avoids the use of drugs administered to combat thrombosis, such as, for example, anti-coagulants, (e.g., heparin), thrombin inhibitors, profibrinolytic agents, warfarin, acenocoumarol, phenprocoumon, atromentin, brodifacoum, phenindone, fondaprinux, idraparinux, argatroban, dabigatran, rivaroxaban, apixaban, which may also target hemostasis. Anti-platelet agents include aspirin, dipyridamole, ticlopidine, clopidogrel, ticagrelor and prasugrel. It is desirable to treat thrombosis without affecting hemostasis to any significant degree. The phrase "significant degree" means that hemostasis is affected to cause abnormal clotting time (prothrombin time) and abnormal platelet function or count.

In some examples anti-coagulation factor therapy involves treating a patient identified to be at risk for premature coagulation with an inhibitor for the coagulation factor or coagulation factors detected. Inhibitors for a coagulation factor include, but are not limited to, small molecule inhibitors for a specific coagulation factor, antibodies for a specific coagulation factor (e.g., factors I to XI), antithrombin, antibodies for a specific coagulation factors, plasmin inhibitors, urokinase inhibitors , tissue factor pathway inhibitors, plasminogen activator inhibitors, and bikunins, for example. Small molecule inhibitor therapy involves administering to a subject identified by methods in accordance with the principles described herein an effective amount of a small molecule inhibitor of a specific coagulation factor present on the CEC. Antibody therapy involves administering to a subject identified by methods in accordance with the principles described herein an effective amount of an antibody specific for an identified coagulation factor present on the CEC.

The phrase "small molecule inhibitor" refers to an inhibitor of a specific coagulation factor that is an organic molecule that has a molecular weight less than about 2000, or less than about 1500, or less than about 1000, or less than about 500, or less than about 400, or less than about 300, for example. In some examples, the small molecule inhibitor has a molecular weight in the range of about 100 to about 2,000, or about 100 to about 1,500, or about 100 to about 1,000, or about 100 to about 500, or about 100 to about 400, or about 100 to about 300, or about 100 to about 200, or about 200 to about 2,000, or about 200 to about 1,500, or about 200 to about 1,000, or about 200 to about 500, or about 200 to about 400, or about 200 to about 300, for example. Small molecule inhibitors for a specific coagulation factor include, by way of illustration and not limitation, direct factor Xa inhibitors (rivaroxaban, apixaban) and direct thrombin inhibitors (hirudin, lepirudin, bivalirudin, argatroban, dabigatran, ximelagatran), for example.

Antibodies against a specific coagulation factor may be prepared using techniques described in detail above for the preparation of antibodies. Antibodies specific for an anticoagulation factor include, but are not limited to, antibodies specific for factor X, antibodies specific for factor IX, antibodies specific for factor VIII, antibodies specific for factor VII, antibodies specific for factor V, antibodies specific for factor IV, antibodies specific for tissue factor, antibodies specific for factor II, antibodies specific for factor I, antibodies specific for thrombin, antibodies specific for plasmin, antibodies specific for prothombin, antibodies specific for antithromin, antibodies specific for thrombomodulin, antibodies specific for plasmin, antibodies specific for plasminogen, antibodies specific for urokinase, antibodies specific for fibronectin, antibodies specific for angiotensin-converting enzyme, specific for Von Willebrand factor (vWF), , antibodies specific for fibrin,, antibodies specific for D-dimer, antibodies specific for tissue plasminogen activator, antibodies specific for protein Z related protease inhibitors, antibodies specific for tissue factor pathway inhibitors, antibodies specific for plasminogen activator inhibitors, antibodies specific for bikunin, antibodies specific for heparin cofactor II, antibodies specific for cancer procoagulant, antibodies specific for protein C, antibodies specific for protein Z and antibodies specific for protein S, for example..

The phrase "at least" as used herein means that the number of specified items may be equal to or greater than the number recited. The phrase "about" as used herein means that the number recited may differ by plus or minus 10%; for example, "about 5" means a range of 4.5 to 5.5.

The following examples further describe the specific embodiments of the invention by way of illustration and not limitation and are intended to describe and not to limit the scope of the invention. Parts and percentages disclosed herein are by volume unless otherwise indicated.

### EXAMPLES

All chemicals may be purchased from the Sigma-Aldrich Company (St. Louis MO) unless otherwise noted.

### Abbreviations:

K₃EDTA = potassium salt of ethylenediaminetetraacetate
WBC = white blood cells
RBC = red blood cells
DAPI = 4',6-diamidino-2-phenylindole
DABCO = 1,4-diazabicyclo[2.2.2]octane
HBSS = Hank's Buffered Salt Solution
min = minute(s)
sec = second(s)
µm = micron(s)
mL = milliliter(s)
mg = milligrams(s)
µg = microgram(s)
PBS = phosphate buffered saline (3.2 mM Na₂HPO₄, 0.5 mM KH₂PO₄,
   1.3 mM KCl, 135 mM NaCl, pH 7.4)
mBar = millibar(s)
RT = room temperature

Whole blood specimens for testing were prepared by collection of blood from 10 subjects containing circulating endothelial cells (CEC). The blood samples (7-10 mL) were collected into VACUTAINER® tubes (Becton, Dickinson and Company, Franklin Lakes NJ) containing K₃EDTA. Additional paraformaldye at 0.05% by volume of the blood sample was added. The concentration of WBC was about 10⁷ per 10 mL blood and of RBC was about 5x10¹⁰ per 10 mL of blood.

Negative and positive control samples were made from whole blood samples by addition of cultured endothelial cells with coagulation factor at a level of 200 cells per 10 mL of blood sample. Cell line primary human umbilical vein endothelial cells (HUVEC) (ATCC PCS-100-010) were grown in ENDOGRO® Basal Media (Millipore Corporation, Billerica MA). Cells were fixed by adding 1.0 mL of 2% formaldehyde HBSS to suspend the cell and incubating at 2°C to 8°C overnight for about 20 h. Coagulation factors, namely Human Factor X, or Tissue Factor or Von Willebrand factor or others were fixed to the cells by means of formaldehyde. Human Factor X, which was purchased from Enzyme Research Laboratory, South Bend IN. All other factors were purchased from Sigma Aldrich. Coagulation factor, Factor X, plays a critical role in initiation of coagulation on TF-bearing cells in the endothelium by amplification of the pro-coagulant signal by thrombin generated on the TF-bearing cell and propagation of thrombin generation on the platelet surface. Both extrinsic and intrinsic pathways converge to Factor Xa. Factor Xa is important as coagulation is activated when Factor Xa is formed on endothelium and CEC in a Xa/Va/Ca/phospholipid complex.

Within one day after storage at 25°C, the blood samples were filtered through a membrane having an average pore size of 8 µm according to a method disclosed in U.S. Patent Application Publication No. 2012/0315. During filtration, the sample on the membrane was subjected to a negative mBar, that is, a decrease greater than about -30 mBar from atmospheric pressure. The vacuum applied varies from 1 to -30 mBar as the volume of the sample reduces from during filtration. High pressure drops were allowable dependent on reservoir and sample volume and filtration rate. Just prior to filtration, a sample (7-10 mL) was transferred to a 50 mL Falcon tube, which was filled to 20 mL with cold PBS. The Falcon tubes were manually overturned twice and subjected to centrifugation for 10 min. at 400 x g at 20°C. The diluted sample was placed into the filtration station without mixing and the diluted sample was filtered through the membrane. Following the filtration, the membrane was washed with PBS, and the sample was fixed with formaldehyde, washed with PBS, subjected to permeabilization using of 0.2% TRITON® X100 in PBS and washed again with PBS.

Cells captured on the membrane were detected with an immunocytochemistry (ICC) procedure based on the binding of specific antibodies to specific proteins or antigens in cells. A blocking buffer of 10% casein in PBS was dispensed on the membrane. After an incubation period of 5 min, the membrane was washed with PBS to block non-specific binding to the membrane. Next, an antibody-conjugate mix was dispensed to the membrane followed by an incubation period of 20 min at RT. The mixture of antibody conjugates (in 10% casein in PBS) included anti-coagulation factor antibody (reactive to either Human Factor X, or Tissue Factor or Von Willebrand factor) conjugated to Dy488 at 10 µg/mL, anti-CEC antibody (reactive to either markers in Table 1) conjugated to Dy550 at 10 µg/mL, and anti-CD45 antibody (used for WBC) conjugated to Dy650 at 20 µg/mL. The mixture of antibody conjugates included anti-endothelial cell antibody (reactive to markers in Table 1) conjugated to Dy550. Unbound antibody was washed away (PBS + 0.05% TWEEN® 20) and DAPI (0.8 µg/mL in PBS), a fluorescent DNA stain was added to stain the nuclei of the cells. A last wash step with PBS was performed, followed by cover media to help preserve the fluorescent intensity of the probes. Slides were made with DABCO as a cover slip medium (0.25g DABCO to 9mL glycerol and 1ml 10 x PBS).

Slides for patients and control were then placed on a slide holder of a fluorescent microscope (Leica DM5000 (Leica Microsystems GmbH, Wetzlar, Germany)) where images were captured during automated scanning of the membrane for each of the fluorescent probes used for detection of targeted cells. Antibody-conjugates became bound to the protein or antigen of a cell, and the fluorescent labels were detected by using a fluorescent microscope with excitation, emission, and cut-off filters specific for each label. Multiple fluorescent labels, each with a different specific antibody, were used to detect multiple antigens or proteins in the isolated cells.

Cells were then characterized by scanning the membrane by fluorescence microscopics conducted with the Leica DM5000 using the filter sets for respective fluorophore labels used in the antibody-conjugates above, namely, DyLight 488 (Dy488), DyLight 550 (Dy550), DyLight 650 (Dy650) (ThermoFisher Scientific, Inc., Waltham MA) or DAPI. Controls were tested first to assign thresholds for detection of positive signals for CEC, WBC and coagulation factor. Enrichment of rare cells achieved was measured by counting the rare cells (CEC positive) and excluding the immune cells (WBC) remaining on the membrane. RBC were either lysed or passed through the membrane.

The results for five different antibodies to markers for CEC, two different antibodies to markers for coagulation factors and one antibody to markers for WBC are summarized below in Table 1. Antibodies were tested against a known native sample containing CEC, CEC activated with coagulation factors and WBC. Antibodies were also tested against a positive control with cultured HUVEC cells fixed with coagulation factors. The signal to noise ratio was assessed as zero to 5+ based on binding to cells in the positive control blood.

**Table 1**

| Marker | Signal to noise Native CEC | for antibody binding Native WBC | by filtration Control HUVEC |
|---|---|---|---|
| Vimentin | ++++ | 0 | + |
| Z0-1 | ++ | 0 | + |
| CD105 | ++ | 0 | ++++ |
| CD144 | + | 0 | + |
| CD146 | +++ | 0 | ++ |
| Anti-Factor X | ++ | 0 | ++++ |
| Anti VWF | ++ | 0 | ++++ |
| CD45 | 0 | +++ | 0 |

All antibodies capable of measuring CEC including vimentin, Z0-1, CD105, CD144, and CD146, were responsive to native CEC and control HUVEC samples with coagulation factors. All antibodies capable of measuring coagulation factors including factor X and vWF, were responsive to CEC activated with coagulation factors and control HUVEC sample with coagulation factors. The CD45 antibody capable of measuring WBC did not respond to CEC activated with coagulation factors or control HUVEC sample with coagulation factors and only to WBC.

In accordance with examples in accordance with the principles described herein, in all cases the CEC and coagulation factors antibodies had surprisingly lower backgrounds in blood samples containing only WBC measured as zero (0). Even antibodies with weak antibody signals (+ to ++) to control cells (lower affinity immunoassay) were able to separate a signal from background noise for native endothelial cell identification. This was an unexpected advantage realized in analyses employing porous matrix filtration techniques and immunocytochemistry techniques. While not wanting to be bound to any particular mechanism, the above advantages are believed to result from the filtration driving the affinity reaction and washing unbound labels more effectively. The CEC cells were counted when the CEC signal was above a threshold and the WBC signal was below a threshold. The threshold was the point at which the signal was detectable over the noise, which in this case was the background signal on the filtration matrix. (See Table 2).

The results for ten healthy and ten unhealthy patients are summarized below in Table 2. The unhealthy patients were those with hyper-activated platelets (hemostasis imbalance). These patients with hyper-activated platelets are more likely to have CEC shedding. Patients with hyper-active platelets were those with closure times below 75 sec when measured with the Collagen/ADP product or below 150 sec when measured with the Collagen/ADP product (PFA-100 Siemens Healthcare Diagnostics, Newark DE). Patients were not on therapy for coagulation for 12 h prior to sample collection.

These patients were tested by different two methods. The first method was employed just to determine the amount of binding signal for CEC by Vimentin antibody labeled with Dy550 and for WBC by CD45 antibody labeled with Dy650, and for coagulation factor by Factor X antibody labeled with Dy448. The relative intensity of signal was judged in two dimension graphs. In one graph, cells in the areas with stronger Dy550 signal and weak Dy650 signal were judged as CEC positive. In another graph, cells in the areas with stronger Dy550 signal and stronger Dy448 signal were judged as activated CEC. This method produced a Dy488, Dy550 and Dy650 signal for each cell and had no cells without a signal. This method represents an approach that would typically be done on a non-filtration cell enrichment method like fluorescence flow cytometry and therefore represents a method not in accordance with the principles described herein and is provided for purposes of comparison. This method did not separate healthy from unhealthy patients very well with significant overlap and produced a percentage of activated CEC in the health population.

The second method was used for filtration cell enrichment to determine the amount of detectable binding threshold for CEC and WBC. This method is a filtration method with highly efficient washing that allowed washing away of background Dy488, Dy550 and Dy650 due to non-specific binding. The method first measured signal for CEC by Vimentin antibody labeled with Dy550 and for WBC by CD45 antibody labeled with Dy650. Only cells with Dy550 signals above the CEC threshold and with Dy650 signals below the WBC threshold are counted as CEC. The coagulation factor signal on the CEC was then measured if above the positive threshold for Dy480 and the CEC were considered activated.

This second method was in accordance with examples in accordance with the principles described herein. The second method separated healthy from unhealthy patients very well without significant overlap and did not produce a percentage of activated CEC in the healthy population. In this second method, a higher percentage of activated CEC in the unhealthy population were detected. The higher the number of CEC, number of activated CEC and the extent of activation, the higher is the correlation to the patient exhibiting premature coagulation. As the coagulation factor signal increased for the CEC, the cells were considered more activated.

**Table 2**

| | Average number of CEC (standard deviation) | | Percentage of CEC with active coagulation factor | |
|---|---|---|---|---|
| Donors | Method 1 | Method 2 | Method 1 | Method 2 |
| Healthy | 8 (10) | 4(4) | 12% | 0% |
| Unhealthy | 20 (19) | 23(8) | 25% | 63% |

| | | | | |
|---|---|---|---|---|
| Method 1 Measured by CEC with Coagulation factor signals without use of predetermined threshold Method 2 Measured by CEC with Coagulation factor signals with use of predetermined threshold | | | | |

Although the foregoing invention has been described in some detail by way of illustration and example for purposes of clarity of understanding, it will be readily apparent to those of ordinary skill in the art in light of the teachings of this invention that certain changes and modifications may be made thereto. Furthermore, the foregoing description, for purposes of explanation, used specific nomenclature to provide a thorough understanding of the invention. However, it will be apparent to one skilled in the art that the specific details are not required in order to practice the invention. Thus, the foregoing descriptions of specific embodiments of the present invention are presented for purposes of illustration and description; they are not intended to be exhaustive or to limit the invention to the precise forms disclosed. Many modifications and variations are possible in view of the above teachings. The embodiments were chosen and described in order to explain the principles of the invention and its practical applications and to thereby enable others skilled in the art to utilize the invention.

## Claims

1. A method of determining a potential of a mammalian subject for exhibiting premature coagulation, the method comprising:
(a) enhancing a concentration of circulating endothelial cells in a sample obtained from the mammalian subject, wherein the concentration of circulating endothelial cells in a sample is carried out by disposing the sample on a side of a porous matrix and applying pressure to the disposed sample,
(b) examining the circulating endothelial cells for the presence of a coagulation factor, and
(c) correlating the presence of the coagulation to the potential of the mammalian subject to exhibit premature coagulation.

2. The method according to claim 1 wherein the sample is a blood sample.

3. The method according to claim 1 wherein the coagulation factor is selected from the group consisting of factors I, II, III tissue factor, IV, V, VI, VII, VIII, IX, X, XI, XI, XII thrombin, prothombin, antithromin, thrombomodulin, plasmin, plasminogen, urokinase, fibronectin, endothelial cell protein C, von Willebrand factor, angiotensin-converting enzyme, fibrin, D-dimer, tissue plasminogen activator, protein Z related protease inhibitor, tissue factor pathway inhibitors, plasminogen activator inhibitor, bikunin, heparin cofactor II, cancer procoagulant, protein C, protein Z and protein S.

4. The method according to claim 1 wherein the pressure applied is about 1 millibar to about 30 millibar and a pore size of the porous matrix is about 1 µm to about 100 µm.

5. The method according to claim 1 wherein concentrated circulating endothelial cells on the porous matrix are examined by immunocytochemistry techniques.

6. The method of claim 1wherein cells are examined for the presence of one or more coagulation factors by exposing the cells to a labeled binding partner for each of the one or more coagulation factors.

7. The method according to claim 1 wherein the concentration of circulating endothelial cells in a sample is enhanced by one or more filtration methods.

## Patentansprüche

1. Verfahren zum Bestimmen eines Potentials eines Säugerindividuums, vorzeitige Gerinnung zu zeigen, wobei das Verfahren Folgendes umfasst:
(a) Verbessern einer Konzentration von zirkulierenden Endothelzellen in einer vom Säugerindividuum erhaltenen Probe, wobei die Konzentration von zirkulierenden Endothelzellen in einer Probe durch Anordnen der Probe auf einer Seite einer porösen Matrix und Anlegen eines Drucks auf die angeordnete Probe durchgeführt wird,
(b) Untersuchen der zirkulierenden Endothelzellen auf das Vorliegen eines Gerinnungsfaktors und
(c) Korrelieren des Vorliegens der Gerinnung mit dem Potential des Säugerindividuums, vorzeitige Gerinnung zu zeigen.

2. Verfahren nach Anspruch 1, wobei es sich bei der Probe um eine Blutprobe handelt.

3. Verfahren nach Anspruch 1, wobei der Gerinnungsfaktor aus der Gruppe bestehend aus Faktoren I, II, III Gewebefaktor, IV, V, VI, VII, VIII, IX, X, XI, XI, XII Thrombin, Prothombin, Antithromin, Thrombomodulin, Plasmin, Plasminogen, Urokinase, Fibronectin, Endothelzellprotein C, Von-Willebrand-Faktor, Angiotensin konvertierendem Enzym, Fibrin, D-Dimer, Gewebe-Plasminogen-Aktivator, mit Protein Z verwandtem Protease-Inhibitor, Gewebefaktor-Weg-Inhibitoren, Plasminogen-Aktivator-Inhibitor, Bikunin, Heparin-Cofaktor II, Krebs-Prokoagulans, Protein C, Protein Z und Protein S ausgewählt ist.

4. Verfahren nach Anspruch 1, wobei der angelegte Druck bei etwa 1 Millibar bis etwa 30 Millibar und eine Porengröße der porösen Matrix bei etwa 1 µm bis etwa 100 µm liegt.

5. Verfahren nach Anspruch 1, wobei die konzentrierten zirkulierenden Endothelzellen auf der porösen Matrix mit immunzytochemischen Techniken untersucht werden.

6. Verfahren gemäß Anspruch 1, wobei Zellen auf das Vorliegen eines oder mehrerer Gerinnungsfaktoren untersucht werden, indem die Zellen mit jeweils einem markierten Bindungspartner für den einen bzw. die mehreren Gerinnungsfaktoren in Kontakt gebracht werden.

7. Verfahren nach Anspruch 1, wobei die Konzentration von zirkulierenden Endothelzellen in einer Probe durch ein oder mehrere Filtrationsverfahren verbessert wird.

## Revendications

1. Procédé de détermination d'un potentiel d'un sujet mammifère à présenter une coagulation prématurée, le procédé comprenant :
(a) l'amélioration d'une concentration de cellules endothéliales en circulation dans un échantillon obtenu à partir du sujet mammifère, dans lequel la concentration de de cellules endothéliales en circulation dans un échantillon est réalisée en disposant l'échantillon sur un côté d'une matrice poreuse et en appliquant une pression à l'échantillon disposé,
(b) l'examen des de cellules endothéliales en circulation pour la présence d'un facteur de coagulation, et
(c) la corrélation de la présence de la coagulation au potentiel du sujet mammifère à présenter une coagulation prématurée.

2. Procédé selon la revendication 1 dans lequel l'échantillon est un échantillon de sang.

3. Procédé selon la revendication 1 dans lequel le facteur de coagulation est sélectionné dans le groupe constitué de facteurs I, II, du facteur tissulaire III, de thrombine IV, V, VI, VII, VIII, IX, X, XI, XI, XII, de prothrombine, d'antithrombine, de plasmine, de plasminogène, d'urokinase, de fibronectine, de protéine de cellules endothéliales C, du facteur Von Willebrand, de l'enzyme de conversion d'angiotensine, de fibrine, de dimère D, de l'activateur de plasminogène tissulaire, de l'inhibiteur de protéase lié à la protéine Z, d'inhibiteurs de voie de facteurs tissulaires, de l'inhibiteur d'activateur de plasminogène, de bikunine, du cofacteur d'héparine II, du pro-coagulant du cancer, de la protéine C, de la protéine Z et de la protéine S.

4. Procédé selon la revendication 1 dans lequel la pression appliquée est d'environ 1 millibar à environ 30 millibars et une taille de pore de la matrice poreuse est d'environ 1 µm à environ 100 µm.

5. Procédé selon la revendication 1 dans lequel des cellules endothéliales en circulation concentrées sur la matrice poreuse sont examinées par des techniques d'immunocytochimie.

6. Procédé selon la revendication 1 dans lequel des cellules sont examinées pour la présence d'un ou de plusieurs facteurs de coagulation en exposant les cellules à un partenaire de liaison étiqueté pour chacun des un ou plusieurs facteurs de coagulation.

7. Procédé selon la revendication 1 dans lequel la concentration de cellules endothéliales en circulation dans un échantillon est améliorée par un ou plusieurs procédés de filtration.
